# EUROPEAN PATENT APPLICATION

(11) **EP 1 947 090 A1**
(43) Date of publication of application: **23.07.2008**
(21) Application number: 07384010.0
(22) Date of filing: 17.01.2007
(51) Int. Cl.: C07D 231/14, C07D 403/14

(54) **Method for preparing N-piperidino-1,5-diphenylpyrazole-3-carboxamide and derivatives**

(71) Applicant: Laboratorios del Dr. Esteve S.A., 08041 Barcelona (ES)
(72) Inventor: Mas Prio, Josep, 08191 Rubi (Barcelona) (ES); Torrens-Jover, Antonio, 08221 Terrassa (Barcelona) (ES)
(74) Representative: Peters, Hajo

(57) **Abstract**

The present invention relates to a method for the preparation of N-Piperidino-1,5-Diphenylpyrazole-3-Carboxamides and their derivatives.

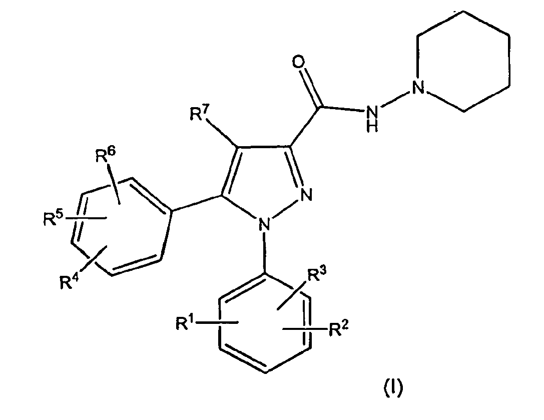

## Description

The present Invention relates to a method for the preparation of N-Piperidino-1,5-Diphenylpyrazole-3-Carboxamides and their derivatives.

Cannabinoids are compounds, which are derived from the cannabis sativa plant which is commonly known as marijuana. The most active chemical compound of the naturally occurring cannabinoids is tetrahydrocannabinol (THC), particularly Δ⁹-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated CB₁ and CB₂ are Involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the CB₁-Receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

N-Piperidino-1,5-Diphenylpyrazole-3-Carboxamides are well known CB1 antagonist, especially exemplified by the 1-H-Pyrazole-3-carboxamide-5(4 chlorophenyl)-1-(2,4-dichlorophenyl)-4-methyl-N-piperidinyl (CAS 168273-06-1) also known as SR141716 or Rimonabant, which is currently marketed under the Trademark "Amplia" for the indication "obesity" in some countries in the world. Even though the compound and processes for its production were described inter alia in EP 656 354 B1 and US 5,624,941, the known production processes for this compound all show some disadvantages, so that - given the possible need for large amounts of the compound - other ways of producing N-Piperidino-1,5-Diphenylpyrazole-3-Carboxamides are needed.

In general It is know that certain compounds of formula general (I) are CB1 modulators (especially known as antagonists or inverse agonists) especially the N-piperidino-5-(4-chlorophenyl)-1-(2,4-dichlorophenyl)-4-methylpyrazole-3-carboxamide and N-piperidino-5-(4-bromophenyl)-1-(2,4-dichlorophenyl)-4-ethylpyrazole-3-carboxamide, N-piperidino-5-(4-hydroxyphenyl)-1-(2,4-dichlorophenyl)-4-methylpyrazole-3-carboxamide, the N-piperidino-5-(4-methoxyphenyl)-1-(2,4-dichlorophenyl)-4-methylpyrazole-3-carboxamide and their salts. These compounds are described in e.g. WO 00/46209; EP 0656354; EP 1150961, WO 2005080343; J.Med.Chem., 2003, 46, 642-645.

Thus, it was an object of the present invention to provide methods for obtaining N-Piperidino-1,5-Diphenylpyrazole-3-Carboxamides, especially Rimonant, for use as active substances in medicaments. In particular, these active substances should be suitable for the modulation of Cannabinoid receptors, more particularly for the modulation of Cannabinoid 1 (CB₁) receptors.

Said object was achieved by providing a method for obtaining the substituted N-Piperidino-1,5-Diphenylpyrazole-3-Carboxamides of general formula I given below, corresponding salts and corresponding solvates thereof.

It has been found that the given production process is a very advantageous e.g. economically and ecologically useful way of producing N-Piperidino-1,5-Diphenylpyrazole-3-Carboxamides and their derivatives, e.g. Rimonabant. In particular this production process possesses the advantages of a simple procedure, mild conditions, and a good yield.

In a preferred aspect the invention thus provides a process for obtaining an N-Piperidino-1,5-diphenylpyrazole-3-carboxamide of general formula (I) 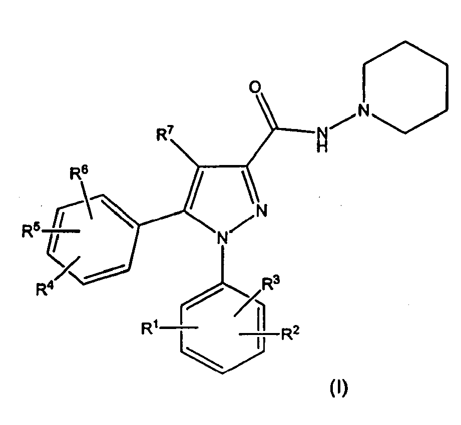 wherein
R¹, R², R³, R⁴, R⁵ and R⁶ independently of each other represent hydrogen; linear or branched C₁₋₆-alkyl, linear or branched C₁₋₆-alkoxy, halogen, CH₂F, CHF₂, CF₃, OH, NO₂, SH, NH₂:
R⁷ represents hydrogen; halogen; linear or branched C₁₋₆-alkyl;
optionally in form of a corresponding salt thereof, or a corresponding solvate thereof;
characterized in that an intermediate compound of general formula II 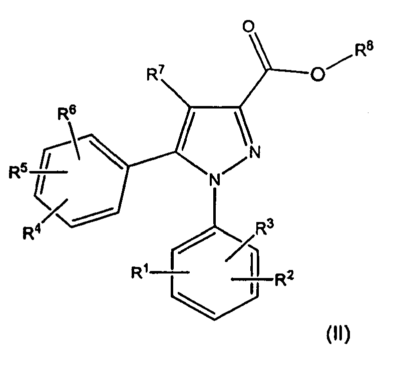 with
R⁸ representing hydrogen; linear or branched C₁₋₆-alkyl;
R¹, R², R³, R⁴, R⁵, R⁶ and R⁷ having the meaning given above; isolated or generated in situ, reacts with hydrazine hydrate, in a suitable reaction medium and suitable reaction conditions; followed by a STEP B, wherein an intermediate compound of general formula III 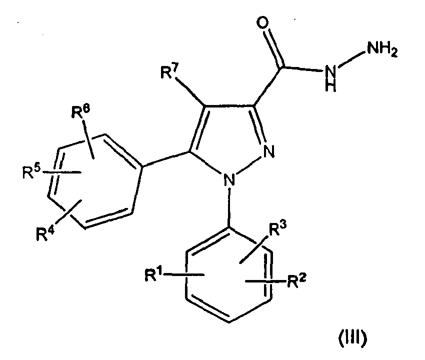 with R¹, R², R³, R⁴, R⁵, R⁶ and R⁷ having the meaning given above; isolated or generated in situ, reacts with benzotriazole and glutaraldehyde in a suitable reaction medium and suitable reaction conditions, followed by a STEP C wherein an intermediate compound of general formula IV 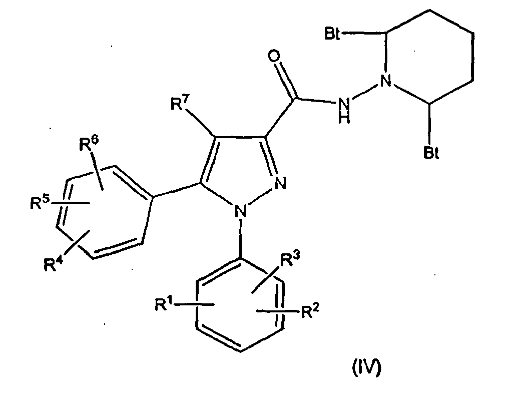 with Bt meaning Benzotriazolyl; and R¹, R², R³, R⁴, R⁵, R⁶ and R⁷ having the meaning given above, isolated or generated in situ, reacts with sodium borohydride in a suitable reaction medium and suitable reaction conditions. In a very preferred embodiment of this process according to the invention
- R¹, R², R³, R⁴, R⁵ and R⁶ independently of each other represent hydrogen; linear or branched C₁₋₆-alkyl, linear or branched C₁₋₆-alkoxy, halogen, CF₃, OH; and/or
- R⁸ represents methyl or ethyl; and/or
- in STEP A the reaction medium is a polar solvent, preferably an alcohol, more preferably EtOH; and/or
- in STEP B the reaction medium is a polar solvent, preferably a water/alcohol mixture, more preferably H₂O/EtOH; and/or
- in STEP C the reaction medium is an organic solvent, preferably THF; and/or
- in STEP A the reaction temperature is first raised to reflux and then cooled down to room temperature; and/or
- in STEP B the reaction temperature is between 0°C and 50 °C, preferably between 15 °C and 40°C, more preferably is room temperature; and/or
- in STEP C the reaction temperature is between 0°C and 50°C, preferably between 15 °C and 40 °C, more preferably is room temperature.

In the context of this invention, "alkyl" is understood as meaning saturated and unsaturated (but not aromatic), branched and linear hydrocarbons, which can be unsubstituted or mono- or polysubstituted. In these radicals, C₁₋₂-alkyl represents C1- or C2-alkyl, C₁₋₃-alkyl represents C1-, C2- or C3-alkyl, C₁₋₄-alkyl represents C1-, C2-, C3- or C4-alkyl, C₁₋₅-alkyl represents C1-, C2-, C3-, C4-, or C5-alkyl, C₁₋₆-alkyl represents C1-, C2-, C3-, C4-, C5- or C6-alkyl, C₁₋₇-alkyl represents C1-, C2-, C3-, C4-, C5-, C6- or C7-alkyl, C₁₋₈-alkyl represents C1-, C2-, C3-, C4-, C5-, C6-, C7- or C8-alkyl, C₁₋₁₀-alkyl represents C1-, C2-, C3-, C4-, C5-, C6-, C7-, C8-, C9- or C10-alkyl and C₁₋₁₈-alkyl represents C1-, C2-, C3-, C4-, C5-, C6-, C7-, C8-, C9-, C10-, C11-, C12-, C13-, C14-, C15-, C16-, C17- or C18-alkyl. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl.

Here, in connection with alkyl - unless expressly defined otherwise - the term substituted in the context of this invention is understood as meaning replacement of at least one hydrogen radical by F, Cl, Br, I, NH₂, SH or OH, "polysubstituted" radicals being understood as meaning that the replacement takes effect both on different and on the same atoms several times with the same or different substituents, for example three times on the same C atom, as in the case of CF₃, or at different places, as in the case of -CH(OH)-CH=CH-CHCl₂. Particularly preferred substituents here are F, Cl and OH.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions. Especially this covers any "physiologically acceptable salt".

The term "physiologically acceptable salt" to be understood as being equivalent and interchangeable with "physiologically acceptable salt" means in the context of this invention any salt that is physiologically tolerated (most of the time meaning not being toxic- especially not caused by the counter-ion) if used appropriately for a treatment especially if used on or applied to humans and/or mammals.
These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this Invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The term "solvate" according to this invention is to be understood as meaning any form of the active compound produced according to the invention in which this compound has attached to it via non-covalent binding another molecule (most likely a polar solvent) especially including hydrates (mono- and multihydrates) and alcoholates, e.g. methanolate and ethanolate.

The term "salt" is to be understood as meaning any form of the active compound produced used according to the Invention in which it assumes an ionic form or is charged and is coupled with a counter-ion (a cation or anion) or is in solution. By this are also to be understood complexes of the active compound produced with other molecules and ions, in particular complexes which are complexed via ionic interactions.

These physiologically acceptable salts can be formed with cations or bases and in the context of this invention is understood as meaning salts of at least one of the compounds produced used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds produced used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

Unless otherwise stated, the compounds produced of the invention are also meant to include compounds produced which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

Solvates, preferably hydrates, of the substituted compounds of general formula (I), of corresponding N-oxides or of corresponding salts thereof may also be obtained by standard procedures known to those skilled in the art.

Substituted pyrazoline compounds produced of general formula I, which comprise nitrogenatom containing saturated, unsaturated or aromatic rings may also be obtained in the form of their N-oxides by methods well known to those skilled in the art.

Those skilled in the art understand that the term substituted pyrazoline compounds as produced herein is to be understood as encompassing derivatives such as ethers, esters and complexes of these compounds as well. The term "derivatives" as used in this application is defined here as meaning a chemical compound having undergone a chemical derivation starting from an acting (active) compound to change (ameliorate for pharmaceutical use) any of its physico-chemical properties, especially a so-called prodrug, e.g. their esters and ethers. Examples of well known methods of producing a prodrug of a given acting compound are known to those skilled in the art and can be found e.g. in Krogsgaard-Larsen et al., Textbook of Drugdesign and Discovery, Taylor & Francis (April 2002). The respective description is hereby incorporated by reference and forms part of the disclosure.

The purification and isolation of the produced substituted compounds of general formula (I) or salt, or N-oxide, or solvate or any intermediate thereof may, if required, be carried out by conventional methods known to those skilled in the art, e.g. chromatographic methods or recrystallization.

In a preferred aspect the invention provides a process for obtaining an N-Piperidino-1,5-diphenylpyrazole-3-carboxamide of general formula (1) 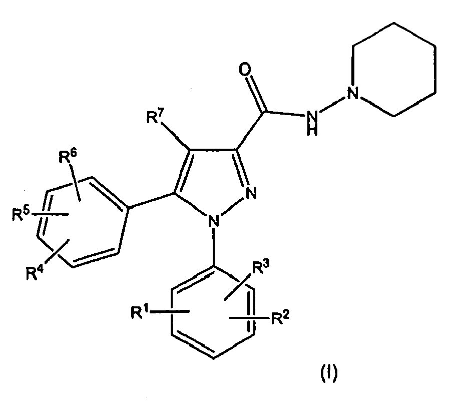 wherein
R¹, R², R³, R⁴, R⁵ and R⁶ independently of each other represent hydrogen; linear or branched C₁₋₆-alkyl, linear or branched C₁₋₆-alkoxy, halogen, CH₂F, CHF₂, CF₃, OH, NO₂, SH, NH₂;
R⁷ represents hydrogen; halogen; linear or branched C₁₋₆-alkyl;
optionally in form of a corresponding salt thereof, or a corresponding solvate thereof;
characterized in that in STEP C an intermediate compound of general formula IV 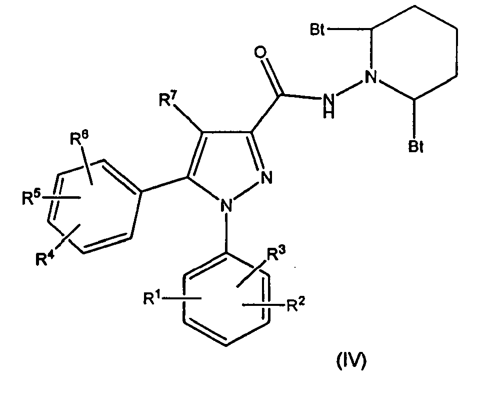 with Bt meaning Benzotriazolyl; and R¹, R², R³, R⁴, R⁵, R⁶ and R⁷ having the meaning given above, isolated or generated in situ, reacts with sodium borohydride in a suitable reaction medium and suitable reaction conditions. In a very preferred embodiment of this process according to the invention
- R¹, R², R³, R⁴, R⁵ and R⁶ independently of each other represent hydrogen; linear or branched C₁₋₆-alkyl, linear or branched C₁₋₆-alkoxy, halogen, CF₃, OH; and/or
- the reaction medium is an organic solvent, preferably THF; and/or
- the reaction temperature is between 0°C and 50 °C, preferably between 15 °C and 40 °C, more preferably is room temperature.

In a preferred aspect the invention provides a process for obtaining a compound according to general formula (IV) 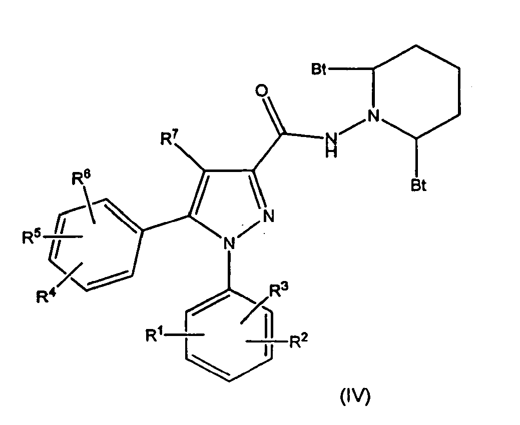 , wherein
R¹, R², R³, R⁴, R⁵ and R⁶ independently of each other represent hydrogen; linear or branched C₁₋₆-alkyl, linear or branched C₁₋₆-alkoxy, halogen, CH₂F, CHF₂, CF₃, OH, NO₂, SH, NH₂;
k⁷ represents hydrogen; halogen; linear or branched C₁₋₆-alkyl;
with Bt meaning Benzotriazolyl;
characterized in that in STEP B an intermediate compound of general formula III 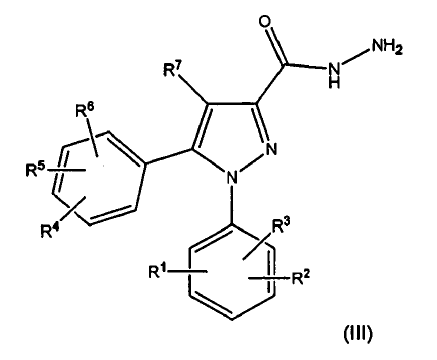 with R¹, R², R³, R⁴, R⁵, R⁶ and R⁷ having the meaning given above; isolated or generated in situ, reacts with benzotriazole and glutaraldehyde in a suitable reaction medium and suitable reaction conditions. In a very preferred embodiment of this process according to the invention
- R¹, R², R³, R⁴, R⁵ and R⁶ independently of each other represent hydrogen; linear or branched C₁₋₆-alkyl, linear or branched C₁₋₆-alkoxy, halogen, CF₃, OH; and/or
- the reaction medium is a polar solvent, preferably a water/alcohol mixture, more preferably H₂O/EtOH; and/or
- the reaction temperature is between 0°C and 50°C, preferably between 15 °C and 40°C, more preferably is room temperature.

In a preferred aspect the invention provides a process for obtaining a compound according to general formula (III) 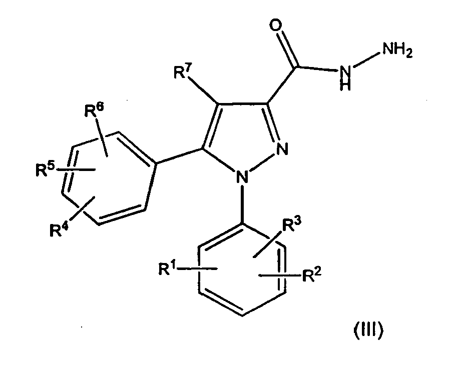 , wherein
R¹, R², R³, R⁴, R⁵ and R⁶ independently of each other represent hydrogen; linear or branched C₁₋₆-alkyl, linear or branched C₁₋₆-alkoxy, halogen, CH₂F, CHF₂, CF₃, OH, NO₂, SH, NH₂;
R⁷ represents hydrogen; halogen; linear or branched C₁₋₆-alkyl;
characterized In that in STEP A a compound of general formula II 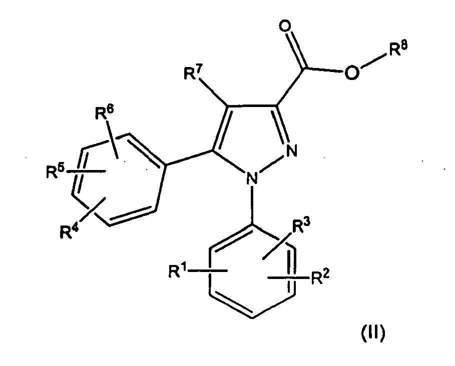 with
R⁸ representing hydrogen; linear or branched C₁₋₆-alkyl;
R¹, R², R³, R⁴, R⁵, R⁶ and R⁷ having the meaning given above; isolated or generated in situ, reacts with hydrazine hydrate, in a suitable reaction medium and suitable reaction conditions. In a very preferred embodiment of this process according to the invention
- R¹, R², R³, R⁴, R⁵ and R⁶ independently of each other represent hydrogen; linear or branched C₁₋₆-alkyl, linear or branched C₁₋₆-alkoxy, halogen, CF₃, OH; and/or
- R⁸ represents methyl or ethyl;
- the reaction medium is a polar solvent, preferably an alcohol, more preferably EtOH; and/or
- the reaction temperature is first raised to reflux and then cooled down to room temperature.

In a preferred aspect the invention provides a process for obtaining a compound according to general formula (IV) 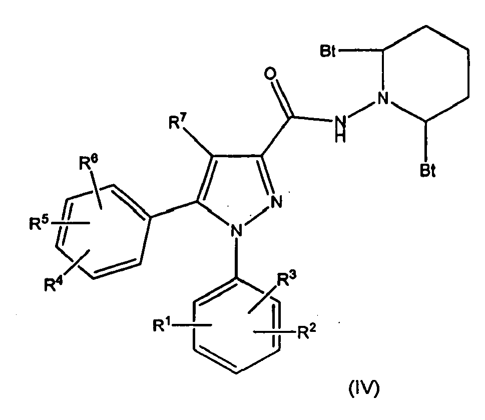 , wherein
R¹, R², R³, R⁴, R⁵ and R⁶ independently of each other represent hydrogen; linear or branched C₁₋₆-alkyl, linear or branched C₁₋₆-alkoxy, halogen, CH₂F, CHF₂, CF₃, OH, NO₂, SH, NH₂;
R⁷ represents hydrogen; halogen; linear or branched C₁₋₆-alkyl;
with Bt meaning Benzotriazolyl;
characterized in that in STEP A a compound of general formula II 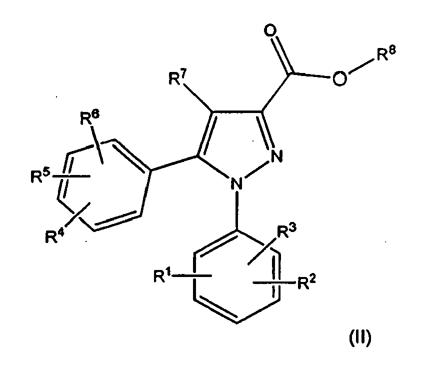 with
R⁸ representing hydrogen; linear or branched C₁₋₆-alkyl
R¹, R², R³. R⁴, R⁵, R⁶ and R⁷ having the meaning given above; isolated or generated in situ, reacts with benzotriazole and glutaraldehyde in a suitable reaction medium and suitable reaction conditions, followed by a STEP B wherein an intermediate compound of general formula III 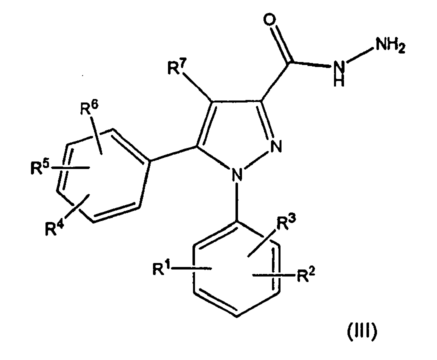 with R¹, R², R³, R⁴, R⁵, R⁶ and R⁷ having the meaning given above; isolated or generated in situ, reacts with benzotriazole and glutaraldehyde in a suitable reaction medium and suitable reaction conditions. In a very preferred embodiment of this process according to the invention
- R¹, R², R³, R⁴, R⁵ and R⁶ independently of each other represent hydrogen; linear or branched C₁₋₆-alkyl, linear or branched C₁₋₆-alkoxy, halogen, CF₃, OH; and/or
- R⁸ represents methyl or ethyl; and/or
- in STEP A the reaction medium is a polar solvent, preferably an alcohol, more preferably EtOH; and/or
- in STEP B the reaction medium is a polar solvent, preferably a water/alcohol mixture, more preferably H₂O/EtOH; and/or
- in STEP A the reaction temperature is first raised to reflux and then cooled down to room temperature; and/or
- in STEP B the reaction temperature is between 0°C and 50 °C, preferably between 15 °C and 40°C, more preferably is room temperature.

In a preferred aspect the Invention provides a process for obtaining an N-Piperidino-1,5-diphenylpyrazole-3-carboxamide of general formula (I) 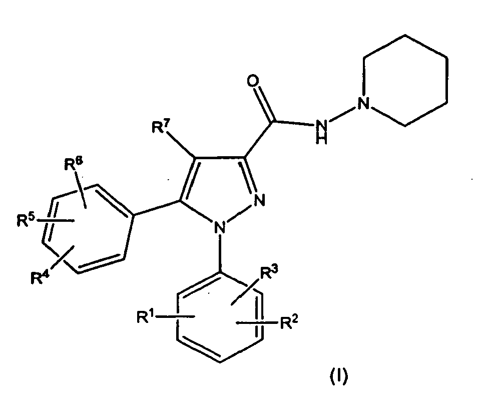 wherein
R¹, R², R³, R⁴, R⁵ and R⁶ independently of each other represent hydrogen; linear or branched C₁₋₆-alkyl, linear or branched C₁₋₆-alkoxy, halogen, CH₂F, CHF₂, CF₃, OH, NO₂, SH, NH₂;
R⁷ represents hydrogen; halogen; linear or branched C₁₋₆-alkyl;
optionally in form of a corresponding salt thereof, or a corresponding solvate thereof;
characterized in that in a STEP B an intermediate compound of general formula III 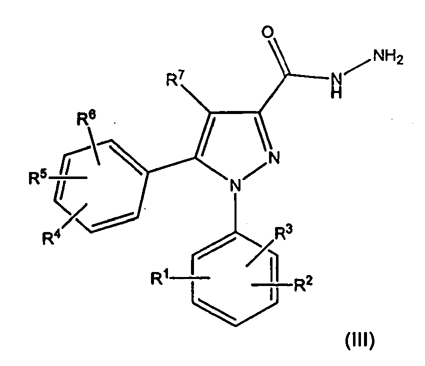 with R¹, R², R³, R⁴, R⁵, R⁶ and R⁷ having the meaning given above; isolated or generated in situ, reacts with benzotriazole and glutaraldehyde in a suitable reaction medium and suitable reaction conditions, followed by a STEP C wherein an intermediate compound of general formula IV 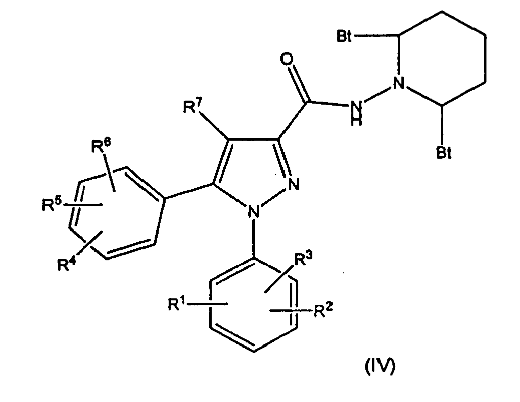 with Bt meaning Benzotriazolyl; and R¹, R², R³, R⁴, R⁵, R⁶ and R⁷ having the meaning given above, isolated or generated in situ, reacts with sodium borohydride in a suitable reaction medium and suitable reaction conditions. In a very preferred embodiment of this process according to the invention
- R¹, R², R³, R⁴, R⁵ and R⁶ independently of each other represent hydrogen; linear or branched C₁₋₆-alkyl, linear or branched C₁₋₆-alkoxy, halogen, CF₃, OH; and/or
- in STEP C the reaction medium is an organic solvent, preferably THF; and/or
- in STEP B the reaction medium is a polar solvent, preferably a water/alcohol mixture, more preferably H₂O/EtOH; and/or
- in STEP C the reaction temperature is between 0°C and 50 °C, preferably between 15 °C and 40 °C, more preferably is room temperature
- in STEP B the reaction temperature is between 0°C and 50 °C, preferably between 15°C and 40 °C, more preferably is room temperature.

In a very preferred embodiment of the process or processes according to the invention
- R¹, R², R³, R⁴, R⁵ and R⁶ independently of each other represent hydrogen; F, Cl, Br, CH₃, C₂H₅, CF₃, OCH₃, OH; preferably hydrogen; OCH₃, Cl, Br, OH.

In a very preferred embodiment of the process or processes according to the invention,
one of R¹, R² and R³ represents hydrogen, the second represents Cl, and the third represents Cl; preferably one of R¹, R² and R³ represents hydrogen, the second represents Cl In ortho-position, and the third represents Cl in para-position; and/or
two of R⁴, R⁵ and R⁶ represent hydrogen, while the third represents Cl, Br,OH or OCH₃; preferably two of R⁴, R⁵ and R⁶ represent hydrogen, while the third represents Cl, Br,OH or OCH₃ in para-position.

In a very preferred embodiment of the process or processes according to the invention
- R⁷ represents hydrogen, CH₃ or C₂H₅, preferably CH₃ or C₂H₅.

In a very preferred embodiment of the process or processes according to the invention, where suitable the compound according to formula I is selected from
- N-piperidino-5-(4-chlorophenyl)-1-(2,4-dichlorophenyl)-4-methylpyrazole-3-carboxamide;
- N-piperidino-5-(4-bromophenyl)-1-(2,4-dichlorophenyl)-4-ethylpyrazole-3-carboxamide,
- N-piperidino-5-(4-hydroxyphenyl)-1-(2,4-dichlorophenyl)-4-methylpyrazole-3-carboxamide, or
- N-piperidino-5-(4-methoxyphenyl)-1-(2,4-dichlorophenyl)-4-methylpyrazole-3-carboxamide and yours salts

A very highly preferred embodiment of the invention is a process according to the invention for obtaining N-Piperidino-1,5-diphenylpyrazole-3-carboxamide of general formula (1a) 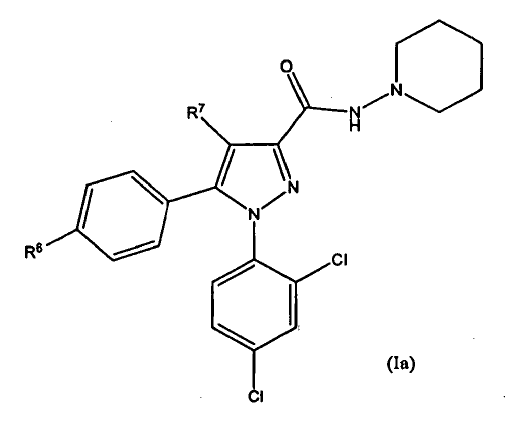 , wherein
R⁶ represents Cl, OH, Br, OCH₃;
R⁷ represents CH₃ or C₂H₅; optionally in form of a corresponding salt thereof, or a corresponding solvate thereof;
characterized in that an intermediate compound of below formula (IIa) 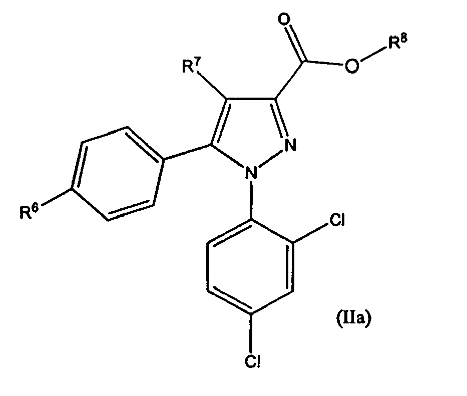 with R⁶ and R⁷ having the meaning given above; R⁸ representing CH₃ or C₂H₅; isolated or generated in situ, reacts with hydrazine hydrate, in a suitable reaction medium and suitable reaction conditions; followed by a STEP B, wherein an intermediate compound of below formula (IIIa) 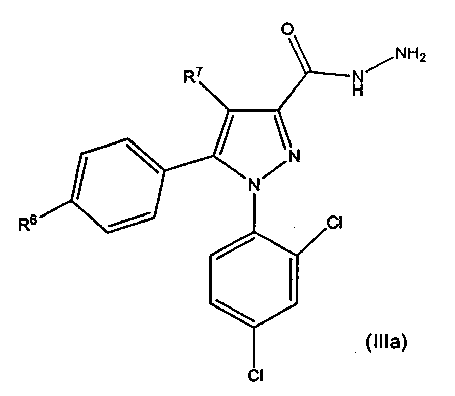 with R⁶ and R⁷ having the meaning given above; isolated or generated in situ, reacts with benzotriazole and glutaraldehyde in a suitable reaction medium and suitable reaction conditions, followed by a STEP C wherein an intermediate compound of below formula IVa 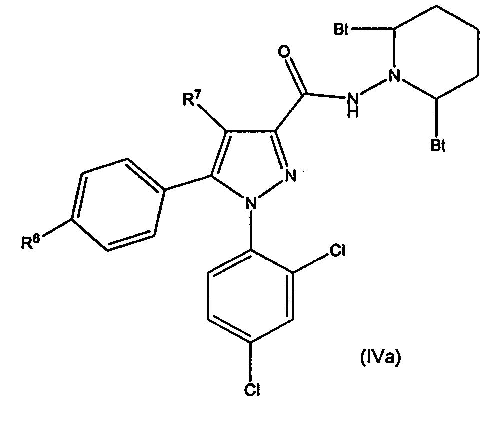 with Bt meaning Benzotriazolyl; and R⁶ and R⁷ having the meaning given above; isolated or generated in situ, reacts with sodium borohydride in a suitable reaction medium and suitable reaction conditions. In a very preferred embodiment of this process according to the invention
- in STEP A the reaction medium is a polar solvent, preferably an alcohol, more preferably EtOH; and/or
- in STEP B the reaction medium is a polar solvent, preferably a water/alcohol mixture, more preferably H₂O/EtOH; and/or
- in STEP C the reaction medium is an organic solvent, preferably THF; and/or
- in STEP A the reaction temperature is first raised to reflux and then cooled down to room temperature; and/or
- in STEP B the reaction temperature is between 0°C and 50 °C, preferably between 15 °C and 40 °C, more preferably is room temperature; and/or
- in STEP C the reaction temperature is between 0°C and 50 °C, preferably between 15 °C and 40 °C, more preferably is room temperature.

The present invention is illustrated below with the aid of examples. These illustrations are given solely by way of example and do not limit the general spirit of the present invention.

### Examples

### General Description:

In general, the substituted 1,5-diphenylpirazole-3-carboxylic acid esters (compounds according to formula (II)), can be obtained as described in e.g. EP 268 554 A1, US 5,114,462.

A general scheme for the production of the N-Piperidino-1,5-diphenylpyrazole-3-carboxamide of general formula (I) is given below:

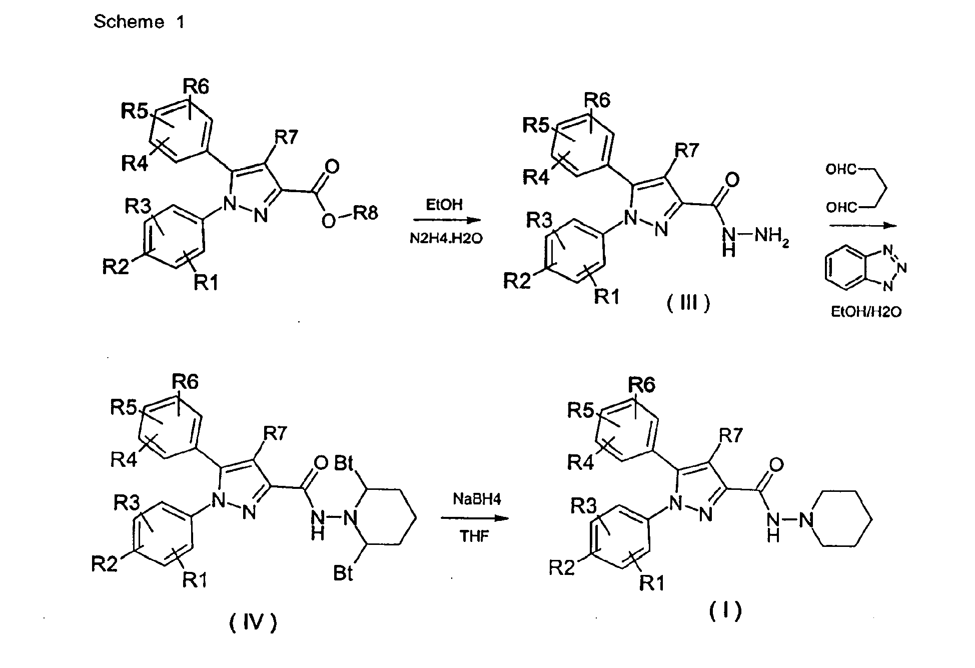

### Example 1

### 5-(4-Chlorophonyl)-1-(2,4-dichlorophonyl)-4-methyl-1H-pyrazole-3-carboxylic acid hydrazide

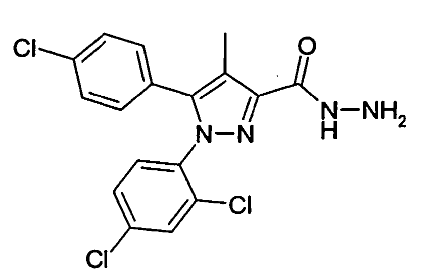

A mixture of 5-(4-Chloro-phenyl)-1-(2,4-dichloro-phenyl)-4-methyl -1 H-pyrazole-3-carboxylic acid ethyl ester (10.70 g, 26 mMol) and 25 g the hydrazine hydrate in ethanol (100 mL), was heated to reflux for 4 hours. The reaction was cooled a room temperature and the ethanol is concentrated in vacuum. To the resulting residue taken up in water (100 mL), filtered and was washed with water, dried in vacuum, yielded the title compound (9,90, 97%) grey solid.
1 H NMR (300 MHz, CHLOROFORM-*d*) δ ppm 2.37 (s, 3 H) 3.02 (br. s., 2 H) 7.06 (d, J=8.50 Hz, 2 H) 7.28 (m, 4 H) 7.43 (d, *J*=1.90 Hz, 1 H)
IR( KBr) 3318, 3133, 1660, 1518, 1496, 1475, 1093, 1091, 970,831cm-1

### Example 2

### 5-(4-Chlorophenyl)-1-(2,4-dichlorophenyl)-4-methyl-1H-pyrazole-3-carboxylic acid(2,6-benzotriazolyl-piperidin-1-yl)amide

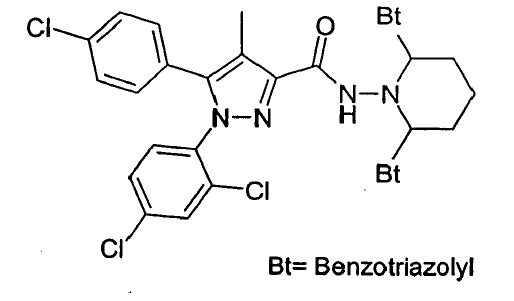

Benzotriazole (238 mg, 2 mMol), the 5-(4-Chloro-phenyl)-1-(2,4-dichloro-phenyl)-4-methyl-1 H-pyrazole-3-carboxylic acid hydrazide (395 mg, 1 mMol) and water (5 mL) and Ethanol (20 mL) were stirred for 10 min at room temperature. Glutaraldehyde ( 400 mg, 1 mMol) 25% aqueous solution, was slowly added to the reaction mixture, and the stirring was continued overnight at,room temperature The ethanol was removed and the insoluble products were filtered off, washed with water, and dried under vacuum, yielded the title compound (390 mg, 56%) grey solid. The spectra of NMR are too complicated to be assigned due the many possible isomers (1 and 2 benzotriazolyl) together with cis and trans forms of 2,6-disubstituted piperidines.

### Example 3

### 5-(4-Chloro-phonyl)-l-(2,4-dichloro-phenyl)-4-mothyl-1H-pyrazole-3-carboxylic acid piperldin-1-ylamide [ this compound may also be referred to as N-piperidino-5-(4-chlorophenyl)-1-(2,4-dichlorophenyl)-4-methylpyrazole-3-carboxamide]

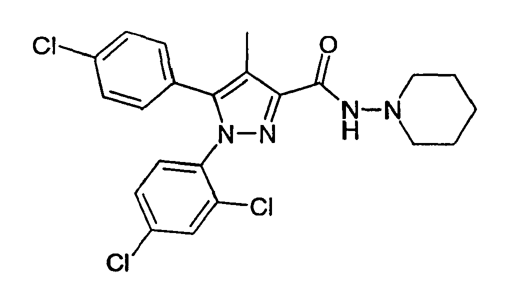

5-(4-Chloro-phenyl)-1-(2,4-dichloro-phenyl)-4-methyl-1 H-pyrazole-3-carboxylic acid(2,6-benzotriazlyl-piperidin-1-yl)amide (382 mg, 0.55 mMol) in THF (15 mL) was stirred at room temperature with sodium borohydride (57 mg, 1,65 mMol) overnight. The reaction was quenched with water, and the product was extracted with dichloromethane washed with water, dried . The solvent evaporated under reduced pressure to obtain the title compound. The product was purified by chromatography with silica gel, eluting with Chloroform/ Methanol system of appropriate polarity, yielded the title compound (1,18g, 46%) white solid.
1 H NMR (300 MHz, CHLOROFORM-*d*) δ ppm 1.53 (br. s., 2 H) 1.90 (br. s., 4 H) 2.35 (s, 3 H) 3.31 (br. s., 4 H) 7.04 (d, *J*=8.50 Hz, 2 H) 7.29 (d, *J*=1.90 Hz, 2 H) 7.31 (d, *J*=1.90 Hz, 1 H) 7.30 (br. s., 1 H) 7.42 (d, *J*=1.76 Hz, 1 H)

## Claims

1. A process for obtaining an N-Piperidino-1,5-diphenylpyrazole-3-carboxamide of general formula (I) 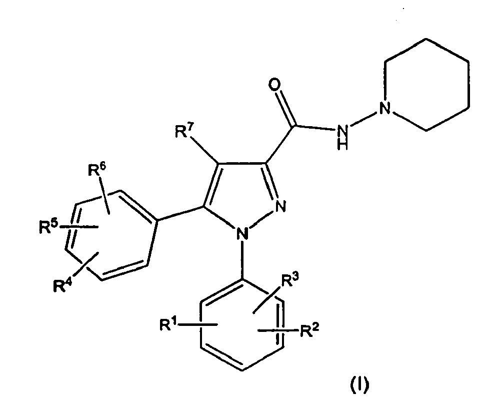 wherein
R¹, R², R³, R⁴, R⁵ and R⁶ independently of each other represent hydrogen; linear or branched C₁₋₆-alkyl, linear or branched C₁₋₆alkoxy, halogen, CH₂F, CHF₂, CF₃, OH, NO₂, SH, NH₂;
R⁷ represents hydrogen; halogen; linear or branched C₁₋₆-alkyl;
optionally in form of a corresponding salt thereof, or a corresponding solvate thereof;
**characterized In that** an intermediate compound of general formula II 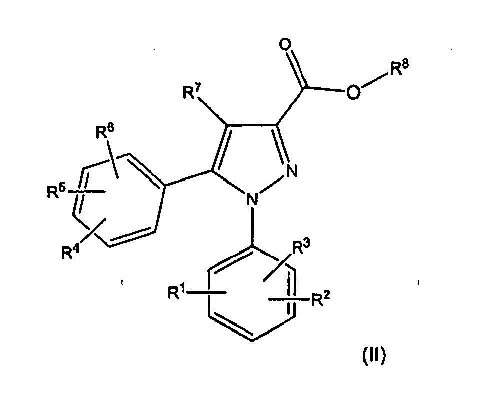 with
R⁸ representing hydrogen; linear or branched C₁₋₆-alkyl;
R¹, R², R³, R⁴, R⁵, R⁶ and R⁷ having the meaning given above; isolated or generated in situ, reacts with hydrazine hydrate, in a suitable reaction medium and suitable reaction conditions; followed by a STEP B, wherein an intermediate compound of general formula III 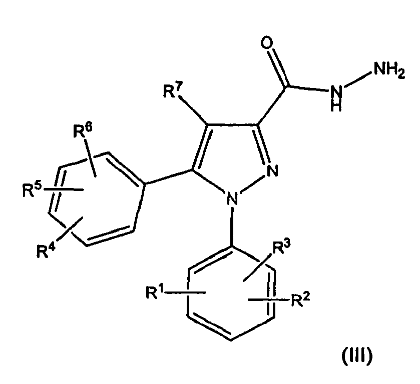 with R¹, R², R³, R⁴, R⁵, R⁶ and R⁷ having the meaning given above; isolated or generated in situ, reacts with benzotriazole and glutaraldehyde in a suitable reaction medium and suitable reaction conditions, followed by a STEP C wherein an intermediate compound of general formula IV 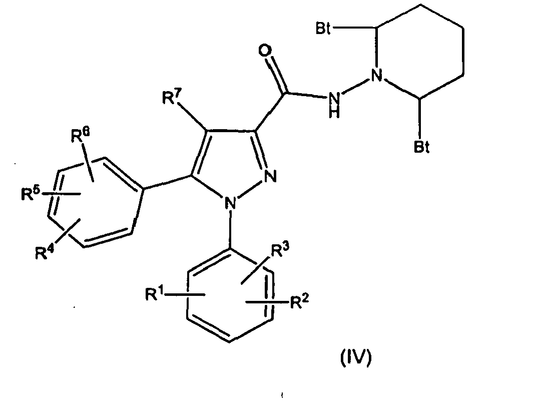 with Bt meaning Benzotriazolyl; and R¹, R², R³, R⁴, R⁵, R⁶ and R⁷ having the meaning given above, isolated or generated in situ, reacts with sodium borohydride in a suitable reaction medium and suitable reaction conditions.

2. A process according to claim 1, **characterized in that**
• R¹, R², R³, R⁴, R⁵ and R⁶ Independently of each other represent hydrogen; linear or branched C₁₋₆-alkyl, linear or branched C₁₋₆-alkoxy, halogen, CF₃, OH; and/or
• R⁸ represents methyl or ethyl; and/or
• in STEP A the reaction medium is a polar solvent, preferably an alcohol, more preferably EtOH; and/or
• in STEP B the reaction medium is a polar solvent, preferably a water/alcohol mixture, more preferably H₂O/EtOH; and/or
• in STEP C the reaction medium is an organic solvent, preferably THF; and/or
• in STEP A the reaction temperature is first raised to reflux and then cooled down to room temperature; and/or
• in STEP B the reaction temperature is between 0°C and 50 °C, preferably between 15 °C and 40 °C, more preferably is room temperature; and/or
• in STEP C the reaction temperature is between 0°C and 50 °C, preferably between 15 °C and 40 °C, more preferably is room temperature.

3. A process for obtaining an N-Piperidino-1,5-diphenylpyrazole-3-carboxamide of general formula (I)
wherein 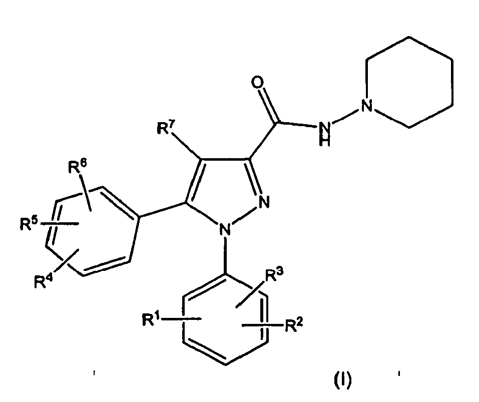 R¹, R², R³, R⁴, R⁵ and R⁶ independently of each other represent hydrogen; linear or branched C₁₋₆-alkyl, linear or branched C₁₋₆-alkoxy, halogen, CH₂F, CHF₂, CF₃, OH, NO₂, SH, NH₂;
R⁷ represents hydrogen; halogen; linear or branched C₁₋₆-alkyl;
optionally in form of a corresponding salt thereof, or a corresponding solvate thereof;
**characterized in that** in STEP C an intermediate compound of general formula IV 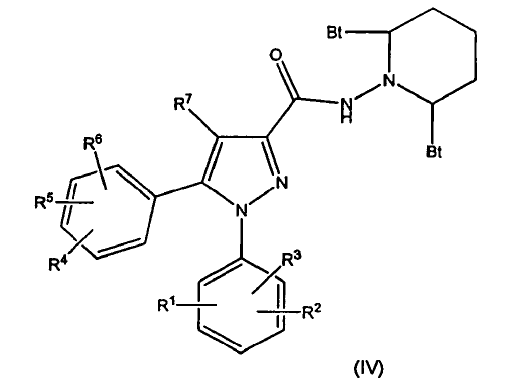 with Bt meaning Benzotriazolyl; and R¹, R², R³, R⁴, R⁵, R⁶ and R⁷ having the meaning given above, isolated or generated in situ, reacts with sodium borohydride in a suitable reaction medium and suitable reaction conditions.

4. A process according to claim 3, **characterized in that**
• R¹, R², R³, R⁴, R⁵ and R⁶ independently of each other represent hydrogen; linear or branched C₁₋₆-alkyl, linear or branched C₁₋₆-alkoxy, halogen, CF₃, OH; and/or
• the reaction medium is an organic solvent, preferably THF; and/or
• the reaction temperature is between 0°C and 50 °C, preferably between 15 °C and 40 °C, more preferably is room temperature.

5. A process for obtaining a compound according to general formula (IV) 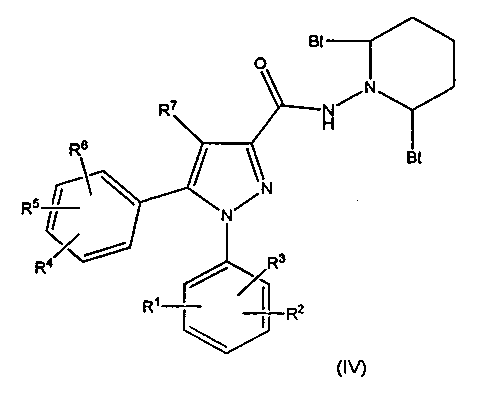 , wherein
R¹, R², R³, R⁴, R⁵ and R⁶ independently of each other represent hydrogen; linear or branched C₁₋₆-alkyl, linear or branched C₁₋₆-alkoxy, halogen, CH₂F, CHF₂, CF₃, OH, NO₂, SH, NH₂:
R⁷ represents hydrogen; halogen; linear or branched C₁₋₆-alkyl;
with Bt meaning Benzotriazolyl;
**characterized in that** in STEP B an intermediate compound of general formula III 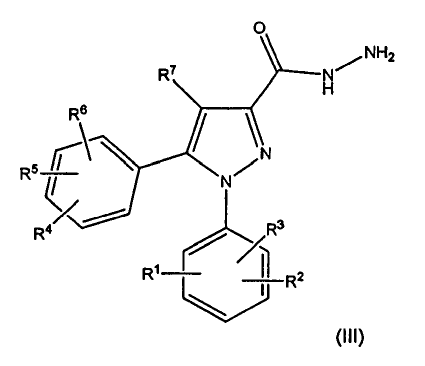 with R¹, R², R³, R⁴, R⁵, R⁶ and R⁷ having the meaning given above; isolated or generated in situ, reacts with benzotriazole and glutaraldehyde in a suitable reaction medium and suitable reaction conditions.

6. A process according to claim 5, **characterized in that**
• R¹, R², R³, R⁴, R⁵ and R⁶ independently of each other represent hydrogen; linear or branched C₁₋₆-alkyl, linear or branched C₁₋₆-alkoxy, halogen, CF₃, OH; and/or
• the reaction medium is a polar solvent, preferably a water/alcohol mixture, more preferably H₂O/EtOH; and/or
• the reaction temperature is between 0°C and 50 °C, preferably between 15 °C and 40 °C, more preferably is room temperature.

7. A process for obtaining a compound according to general formula (III) 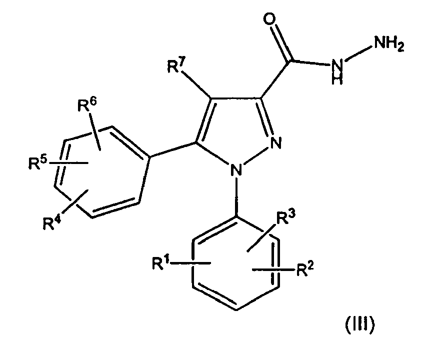 , wherein
R¹, R², R³, R⁴, R⁵ and R⁶ independently of each other represent hydrogen; linear or branched C₁₋₆-alkyl, linear or branched C₁₋₆-alkoxy, halogen, CH₂F, CHF₂, CF₃, OH, NO₂, SH, NH₂;
R⁷ represents hydrogen; halogen; linear or branched C₁₋₆-alkyl;
**characterized in that** in STEP A a compound of general formula II 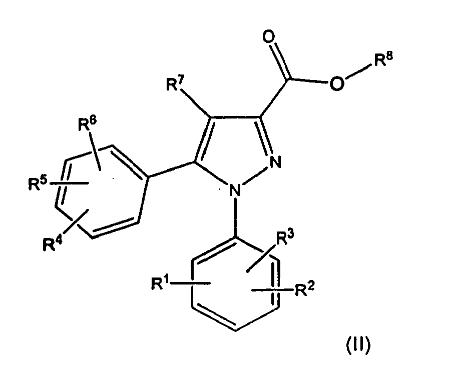 with
R⁸ representing hydrogen; linear or branched C₁₋₆-alkyl;
R¹, R², R³, R⁴, R⁵, R⁶ and R⁷ having the meaning given above; isolated or generated in situ, reacts with hydrazine hydrate, in a suitable reaction medium and suitable reaction conditions.

8. A process according to claim 7, **characterized in that**
• R¹, R², R³, R⁴, R⁵ and R⁶ independently of each other represent hydrogen; linear or branched C₁₋₆-alkyl, linear or branched C₁₋₆-alkoxy, halogen, CF₃, OH; and/or
• R⁸ represents methyl or ethyl;
• the reaction medium is a polar solvent, preferably an alcohol, more preferably EtOH; and/or
• the reaction temperature is first raised to reflux and then cooled down to room temperature.

9. A process for obtaining a compound according to general formula (IV) 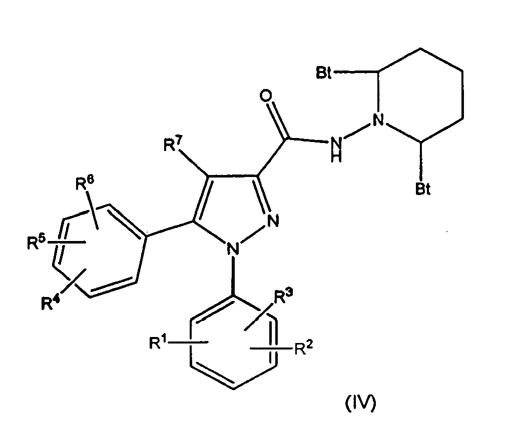 , wherein
R¹, R², R³, R⁴, R⁵ and R⁶ independently of each other represent hydrogen; linear or branched C₁₋₆-alkyl, linear or branched C₁₋₆-alkoxy, halogen, CH₂F, CHF₂, CF₃, OH, NO₂, SH, NH₂;
R⁷ represents hydrogen; halogen; linear or branched C₁₋₆-alkyl;
with Bt meaning Benzotriazolyl;
**characterized in that** in STEP A a compound of general formula II 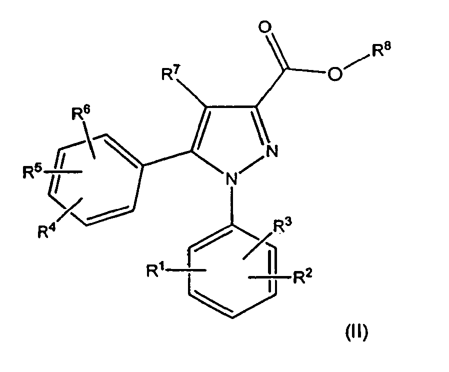 with
R⁸ representing hydrogen; linear or branched C₁₋₆-alkyl
R¹, R², R³, R⁴, R⁵, R⁶ and R⁷ having the meaning given above;
Isolated or generated in situ, reacts with benzotriazole and glutaraldehyde in a suitable reaction medium and suitable reaction conditions, followed by a STEP B wherein an intermediate compound of general formula III 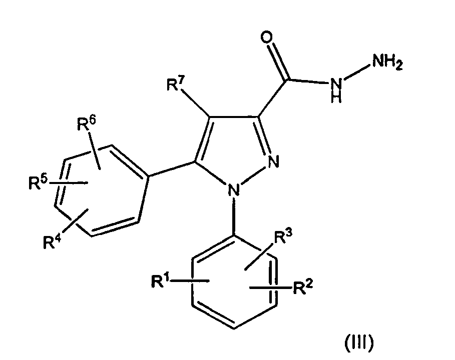 with R¹, R², R³, R⁴, R⁵, R⁶ and R⁷ having the meaning given above; isolated or generated in situ, reacts with benzotriazole and glutaraldehyde in a suitable reaction medium and suitable reaction conditions.

10. A process according to claim 9, **characterized in that**
• R¹, R², R³, R⁴, R⁵ and R⁶ independently of each other represent hydrogen; linear or branched C₁₋₆-alkyl, linear or branched C₁₋₆-alkoxy, halogen, CF₃, OH; and/or
• R⁸ represents methyl or ethyl; and/or
• in STEP A the reaction medium is a polar solvent, preferably an alcohol, more preferably EtOH; and/or
• in STEP B the reaction medium is a polar solvent, preferably a water/alcohol mixture, more preferably H₂O/EtOH; and/or
• in STEP A the reaction temperature is first raised to reflux and then cooled down to room temperature; and/or
• in STEP B the reaction temperature is between 0°C and 50 °C, preferably between 15 °C and 40 °C, more preferably is room temperature.

11. A process for obtaining an N-Piperidino-1,5-diphenylpyrazole-3-carboxamide of general formula (I) 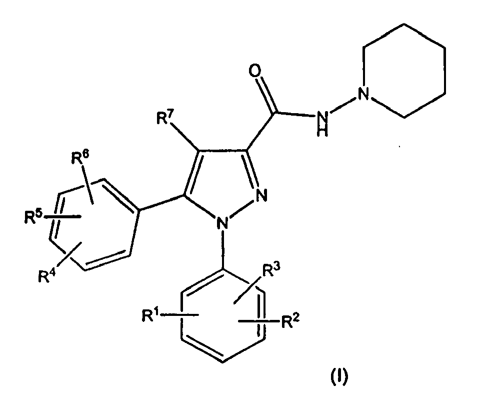 wherein
R¹, R², R³, R⁴, R⁵ and R⁶ independently of each other represent hydrogen; linear or branched C₁₋₆-alkyl, linear or branched C₁₋₆-alkoxy, halogen, CH₂F, CHF₂, CF₃, OH, NO₂, SH, NH₂;
R⁷ represents hydrogen; halogen; linear or branched C₁₋₆-alkyl;
optionally in form of a corresponding salt thereof, or a corresponding solvate thereof;
**characterized in that** in a STEP B an intermediate compound of general formula III 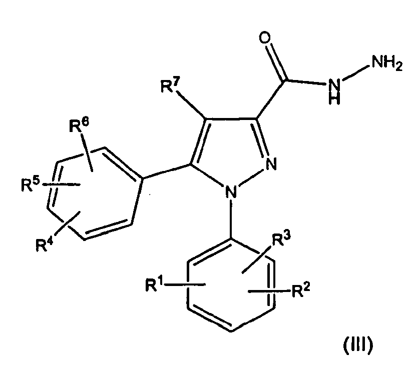 with R¹, R², R³, R⁴, R⁵, R⁶ and R⁷ having the meaning given above; isolated or generated in situ, reacts with benzotriazole and glutaraldehyde in a suitable reaction medium and suitable reaction conditions, followed by a STEP C wherein
an intermediate compound of general formula IV 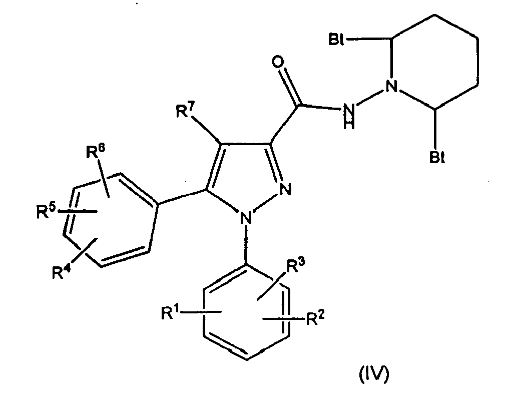 with Bt meaning Benzotriazolyl; and R¹, R², R³, R⁴, R⁵, R⁶ and R⁷ having the meaning given above, isolated or generated in situ, reacts with sodium borohydride in a suitable reaction medium and suitable reaction conditions.

12. A process according to claim 11, **characterized in that**
• R¹, R², R³, R⁴, R⁵ and R⁶ independently of each other represent hydrogen; linear or branched C₁₋₆-alkyl, linear or branched C₁₋₆-alkoxy, halogen, CF₃, OH; and/or
• in STEP C the reaction medium is an organic solvent, preferably THF; and/or
• in STEP B the reaction medium is a polar solvent, preferably a water/alcohol mixture, more preferably H₂O/EtOH; and/or
• in STEP C the reaction temperature is between 0°C and 50 °C, preferably between 15 °C and 40 °C, more preferably is room temperature
• in STEP B the reaction temperature is between 0°C and 50 °C, preferably between 15 °C and 40 °C, more preferably is room temperature.

13. A process according to claims 1, 3, 5, 7, 9 and 11, **characterized in that**
• R¹, R², R³, R⁴, R⁵ and R⁶ independently of each other represent hydrogen; F, Cl, Br, CH₃, C₂H₅, CF₃, OCH₃, OH; preferably hydrogen; OCH₃, Cl, Br, OH.

14. A process according to claims 1, 3, 5, 7, 9 and 11, **characterized in that**
one of R¹, R² and R³ represents hydrogen, the second represents Cl, and the third represents Cl; preferably one of R¹, R² and R³ represents hydrogen, the second represents Cl in ortho-position, and the third represents Cl in para-position; and/or
two of R⁴, R⁵ and R⁶ represent hydrogen, while the third represents Cl, Br,OH or OCH₃; preferably two of R⁴, R⁵ and R⁶ represent hydrogen, while the third represents Cl, Br,OH or OCH₃ in para-position.

15. A process according to any of claims 1 to 13, **characterized in that**
• R⁷represents hydrogen, CH₃ or C₂H₅, preferably CH₃ or C₂H₅.

16. A process according to claim 1, 3, and 11, **characterized in that** the compound according to formula I is selected from
• N-piperidino-5-(4-chlorophenyl)-1-(2,4-dichlorophenyl)-4-methylpyrazole-3-carboxamide;
• N-piperidino-5-(4-bromophenyl)-1-(2,4-dichlorophenyl)-4-ethylpyrazole-3-carboxamide,
• N-piperidino-5-(4-hydroxyphenyl)-1-(2,4-dichlorophenyl)-4-methylpyrazole-3-carboxamide, or
• N-piperidino-5-(4-methoxyphenyl)-1-(2,4-dichlorophenyl)-4-methylpyrazole-3-carboxamide and yours salts

17. A process according to claim 1 for obtaining N-Piperidino-1,5-diphenylpyrazole-3-carboxamide of general formula (Ia) 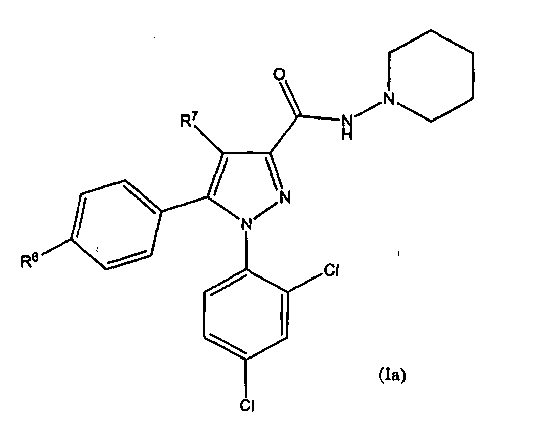 , wherein
R⁶ represents Cl, OH, Br, OCH₃;
R⁷ represents CH₃ or C₂H₅;
optionally in form of a corresponding salt thereof, or a corresponding solvate thereof;
**characterized in that** an intermediate compound of below formula (IIa) 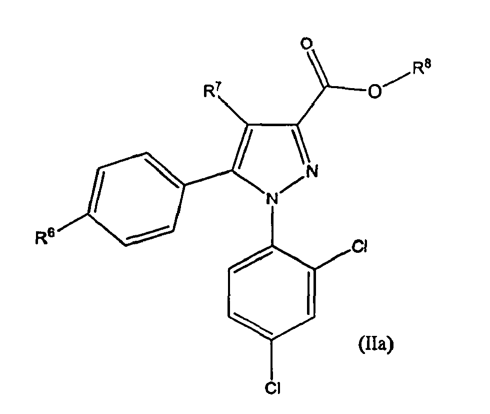 with R⁶ and R⁷ having the meaning given above; R⁸ representing CH₃ or C₂H₅; isolated or generated in situ, reacts with hydrazine hydrate, in a suitable reaction medium and suitable reaction conditions; followed by a STEP B, wherein an intermediate compound of below formula (IIIa) 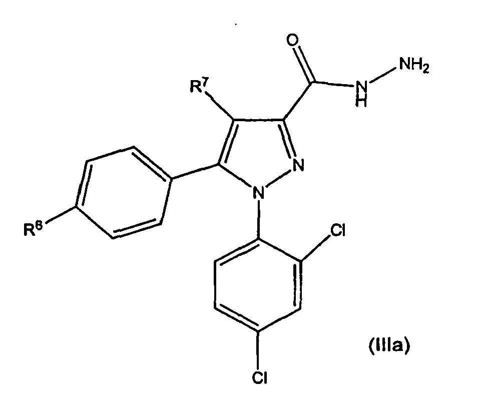 with R⁶ and R⁷ having the meaning given above; isolated or generated in situ, reacts with benzotriazole and glutaraldehyde in a suitable reaction medium and suitable reaction conditions, followed by a STEP C wherein
an intermediate compound of below formula IVa 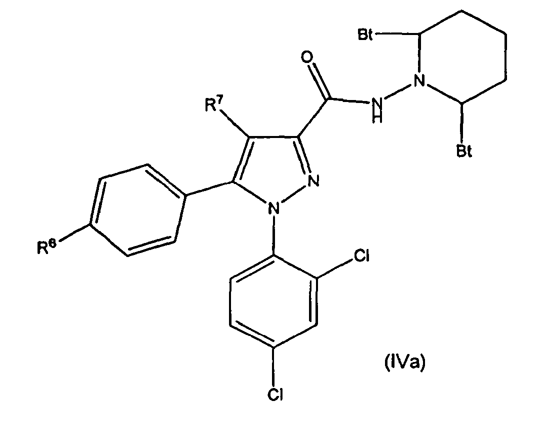 with Bt meaning Benzotriazolyl; and R⁶ and R⁷ having the meaning given above; isolated or generated in situ, reacts with sodium borohydride in a suitable reaction medium and suitable reaction conditions.

18. A process according to claim 17, **characterized in that**
• in STEP A the reaction medium is a polar solvent, preferably an alcohol, more preferably EtOH; and/or
• in STEP B the reaction medium is a polar solvent, preferably a water/alcohol mixture, more preferably H₂O/EtOH; and/or
• in STEP C the reaction medium is an organic solvent, preferably THF; and/or
• in STEP A the reaction temperature is first raised to reflux and then cooled down to room temperature; and/or
• in STEP B the reaction temperature is between 0°C and 50 °C, preferably between 15 °C and 40 °C, more preferably is room temperature; and/or
• in STEP C the reaction temperature is between 0°C and 50 °C, preferably between 15 °C and 40 °C, more preferably is room temperature.
